Europäisches Patentamt

European Patent Office

Office européen des brevets

(11) Publication number: **0 501 233 A1**

## EUROPEAN PATENT APPLICATION

(21) Application number: **92102373.5**

(22) Date of filing: **13.02.92**

(51) Int. Cl.⁵: **C12P  21/08**, C12N 5/20, A61K 39/395, C07K 15/00

(30) Priority: **26.02.91 US 661583**

(43) Date of publication of application:
**02.09.92 Bulletin  92/36**

(84) Designated Contracting States:
**AT BE CH DE DK ES FR GB GR IT LI LU MC NL PT SE**

(71) Applicant: **MILES INC.**
**One Mellon Center 500 Grant Str.**
**Pittsburgh, PA 15219-2502(US)**

(72) Inventor: **Aune, Thomas M.**
**60 Middle Road**
**Hamden, Connecticut 06517(US)**
Inventor: **Rothman-Sabia, Barbara L.**
**226 Skinner Lane**
**Hebron, Connecticut 06248(US)**

(74) Representative: **Dänner, Klaus, Dr. et al**
**c/o Bayer AG Konzernverwaltung RP Patente**
**Konzern**
**W-5090 Leverkusen 1 Bayerwerk(DE)**

(54) **Hybridomas and monoclonal antibodies that inhibit anti-CD3-stimulated T cell proliferation.**

(57)  The present invention relates to antibodies that are immunologically reactive with a T cell surface antigen, CD44. Specifically, the invention relates to monoclonal antibodies, including mAb 212.3, that are immunologically reactive with CD44 and that are able to inhibit OKT3-induced T cell proliferation. The invention also relates to the therapeutic use of such antibodies, including mAb 212.3, and to hybridoma cell lines that produce such antibodies. The invention also relates to an epitope that is comprised of sequences and/or a conformation of sequences present in the CD44 molecule. This epitope is immunologically reactive to mAb 212.3.

EP 0 501 233 A1

## BACKGROUND OF THE INVENTION

### Field of the Invention

This invention relates to hybridomas and monoclonal antibodies that are immunologically reactive with CD44 and anti-CD44 antibodies according to the present invention inhibit the OKT3-induced proliferation of T cells. The invention also relates to epitopes of the CD44 antigen that are immunologically reactive with the monoclonal antibody provided by the invention, and to methods for using these antibodies therapeutically.

### Background of the Related Art

A variety of cell-surface antigenic molecules with molecular weight of 80-100 kilodaltons (kD) have been described previously. These antigens include the Hermes lymphocyte homing receptor (Picker *et al.*, 1989, J. Immunol. 142: 2046), the Pgp-1 molecule (Omary *et al.*, 1988, Immunogenetics 27: 460), HUTCH-1 and the extracellular matrix receptor proteins (Gallatin *et al.*, 1989, Proc. Natl. Acad. Sci. USA 86: 4654). These various antigens have been identified and characterized by a variety of antibodies. (*See*, e.g., Cobbold *et al.*, 1987, in *Leukocyte Typing III: White Cell Differentiation Antigens*, A.J. Michael, ed., Oxford Univ. Press, Oxford, England, p. 788.) As the references cited below indicate, the relationships between these antigens were not initially understood and thus the nomenclature has been confusing. Recently, however, comparisons of immunological cross-reactivity of the antigens recognized by these different antibodies (*see*, e.g., Picker *et al.*, *supra*) as well as comparisons of the cDNA sequences encoding some of these antigens (*see*, e.g., Stamenkovic *et al.*, 1989, Cell 56: 1057 and Goldstein *et al.*, 1989, Cell 56: 1063) have indicated that these antigens are closely related, if not identical, and they have been termed CD44. (*See*, e.g., Haynes *et al.*, 1989, Immunology Today 10: 423 for a review.)

Haynes *et al.*, 1983, J. Immunol. 131: 1195 describe the production of a monoclonal antibody (mAb) AIG3 that is immunologically reactive with an 80 kD cell surface antigen which was mapped to human chromosome 11.

Picker *et al.*, *supra*, disclose and compare a number of independently derived monoclonal antibodies, including Hermes-1, Hermes-2, Hermes-3, H2-7 and H3-51 against the human Hermes antigens, mAb IM7 against the human Pgp-1 antigen, and mAb A1G3 (*see* Haynes *et al.*, 1983, *supra*) and A3D8 against the human *In(Lu)*-related p80 antigen (CD44), all of which react with a common molecular class (CD44/Pgp-1/Hermes) of 85-95 kD lymphocyte surface molecules.

Jalkanen *et al.*, 1987, J. Cell. Biol. 105: 983 disclose that anti-human mAb Hermes-1 and Hermes-3, as well as anti-mouse mAb MEL14, are immunologically reactive to an 85-95 kD antigen that mediates tissue-specific homing of lymphocytes to high endothelial venules (HEV); these antibodies differentially inhibit lymphocyte binding to lymph node, mucosal, or synovial endothelial cells.

Gallatin *et al.*, *supra*, disclose that the monoclonal antibodies P1G12 and P3H9, which are immunologically reactive with the 90 kD extracellular matrix receptor III protein, are immunologically cross-reactive with the antigens recognized by antibodies against human Hermes and macaque HUTCH1 antigens.

Stamenkovic *et al.*, *supra*, disclose the isolation and sequence of a cDNA clone encoding CDw44 from libraries prepared from U937, JY, Raji and KG-1 cell lines.

Goldstein *et al.*, *supra*, disclose the cloning and sequencing of the cDNA for gp90^Hermes (CD44) from a mucosal HEV-binding B lymphoblastoid cell line, KCA. The reported cDNA sequence is identical to the cDNA sequence disclosed by Stamenkovic *et al*, *supra*.

The CD44 molecule was originally described by Dalchau *et al.*, 1980, Eur. J. Immunol. 10: 745 as a human molecule defined by a monoclonal antibody F10-44-2. This molecule was shown by Dalchau *et al.*, *supra*, and Flanagan *et al.*, 1989, Immunology 67: 167, to be present on T cells, granulocytes, cells within the brain and cortical thymocytes. It is now known that the CD44 molecule is a highly glycosylated cell surface serine phosphoprotein expressed by a variety of cell types, including B and T lymphocytes, monocytes, granulocytes, erythrocytes, fibroblasts and keratinocytes and in brain tissue. (*See*, e.g., Isake *et al.*, 1986, Immunogenetics 23: 326 and Haynes *et al.*, 1989, *supra*, for review.) CD44 has been shown to play a role in lymphocyte activation. Anti-CD44 antibodies block binding of lymphocytes to high endothelial venules, inhibit T cell-erythrocyte rosetting, and augment T cell proliferation induced by the CD2 or CD3 T cell receptor pathways. In contrast, anti-CD44 mAbs have no effect on mitogen-stimulated T cell proliferation.

Huet *et al.*, 1989, J. Immunol. 143: 798 disclose the preparation of mAb H90, which is immunologically reactive to CD44. Binding of this antibody to CD44 strongly increases [3H]-thymidine (TdR) incorporation of peripheral blood lymphocytes (PBL) stimulated in combination with either anti-CD2 or anti-CD3 antibodies; such binding has no effect on [3H]-TdR incorporation of PBL stimulated with lectins.

Shimizu *et al.*, 1989, J. Immunol. 143: 2457 disclose the isolation of mAb NIH 44-1; this mAb partially inhibits E-type rosetting of T cells. Binding of this antibody to CD44 strongly increases [3H]-TdR incorporation of PBL stimulated in combination with either anti-CD2 or anti-CD3 antibodies.

Denning *et al.*, 1990, J. Immunol. 144: 7 disclose that CD44 binding with antibodies A3D8 and A1G3 enhance the T cell response to anti-CD2 and anti-CD3 antibodies, and have no effect on T cell proliferation induced by lectin mitogens. Antibody binding increased the interleukin 2 (IL-2) production by T cells.

The present invention relates to the production of novel anti-CD44 antibodies, exemplified by mAb 212.3. Like other anti-CD44 antibodies described in the prior art, antibodies such as mAb 212.3 according to the present invention recognize CD44 expressed at the cell surface of T cells, augment the response of PBL to anti-CD2 antibodies and have no effect on T cell proliferation induced by treatment with lectins. In surprising contrast to other anti-CD44 antibodies known in the prior art, binding of the epitope recognized by antibodies such as mAb 212.3 according to the present invention completely inhibits T cell proliferation via the anti-CD3 pathway. In addition, binding of mAb 212.3 also completely inhibits the production and release of IL-2 associated with anti-CD3-stimulated T cell proliferation. Binding of this antibody also inhibits anti-CD3 induced expression of the IL-2 receptor on the surface of T cells, and inhibits the increase in intracellular calcium ion concentration induced by anti-CD3 antibody. Although one antibody termed L129 has been reported to inhibit anti-CD3 T cell proliferation, this antibody, disclosed in European Patent Application, publication number 0322254A2 published June 28, 1989, is distinguished from antibodies according to the present invention by numerous properties. L129 inhibits T cell proliferation stimulated by lectins as well as T cell proliferation stimulated by anti-CD2 antibodies and by anti-CD3 antibodies, whereas antibodies such as mAb 212.3 according to the present invention have no effect on lectin-stimulated T cell proliferation. In addition, the pattern of expression of the cognate antigen or epitope recognized by the L129 antibody is distinct from the pattern of expression of the cognate antigen or epitope recognized by antibodies according to the present invention, such as mAb 212.3. An important distinction between the specificity of the L129 in contrast to the specificity of anti-CD44 antibodies according to the present invention is illustrated by the fact that expression of the antigen or epitope recognized by L129 is increased on activated T cells, whereas the antigen or epitope recognized by antibodies according to the present invention such as mAb 212.3 is homogeneously expressed on both resting and activated T cells.

The novel and unique features of novel anti-CD44 antibodies according to the present invention, as illustrated by mAb 212.3, distinguish these antibodies from any known in the prior art, and result in novel and useful properties, including therapeutic properties, which are among the objects of the present invention.

## BRIEF DESCRIPTION OF THE FIGURES

Figure 1A illustrates the results of immunoprecipitation of CD44 and Figure 1B illustrates the results of peptide mapping of CD44.

Figure 2 represents the results of flow microfluorimetry analysis of peripheral blood lymphocytes reacted with anti-CD44 mAb.

Figure 3 illustrates the effect of anti-CD44 mAb on OKT3-stimulated T cell proliferation.

Figure 4 illustrates the effect of anti-CD44 mAb on CD2 receptor-stimulated T cell proliferation.

Figure 5 illustrates the effects of anti-CD44 mAb on IL-2 production by T cells stimulated with OKT3.

Figure 6 represents the results of flow microfluorimetry analysis of the effect of anti-CD44 mAb on IL-2 receptor expression using OKT3-stimulated T cells.

Figure 7 illustrates the effect of anti-CD44 mAb on OKT3-induced increases in intracellular calcium ion levels in T cells.

Figure 8 represents the results of flow microfluorimetry analysis of the effect of anti-CD44 mAb on the binding of OKT3 on T cells.

## SUMMARY OF THE INVENTION

The present invention relates to antibodies that are immunologically reactive to a cell-surface antigen of T lymphocytes ("T cells"). The invention relates to a particular cell-surface antigen that is CD44. Specifically, the invention relates to a monoclonal antibody that is immunologically reactive with CD44 and that is able to inhibit OKT3-induced T cell proliferation. The invention also relates to the therapeutic use of such an antibody, and hybridoma cell lines that produce such an antibody. A preferred embodiment of the present invention is a particular monoclonal antibody that is mAb 212.3. The invention also relates to an epitope that is comprised of sequences and/or a conformation of sequences present in the CD44 molecule. This epitope is immunologically reactive to mAb 212.3.

It is an object of the present invention to provide a monoclonal antibody that is immunologically reactive to a cell-surface glycoprotein that is CD44. It is a particular object of the invention to provide an antibody which inhibits OKT3-induced T cell proliferation. In a preferred embodiment, the monoclonal antibody is mAb 212.3.

It is also an object of the present invention to provide a hybridoma cell line that produces such an antibody. It is a particular object of the invention to provide a hybridoma cell line that is the result of fusion between a non-immunoglobulin producing mouse myeloma cell line and spleen cells derived from a mouse immunized with a human cell line which expressed the CD44 antigen. The present invention also provides a hybridoma cell line that produces such an antibody, and that can be injected into a living mouse to provide an ascites fluid from the mouse that is comprised of such an antibody. In a preferred embodiment, the antibody produced by this cell line is mAb 212.3.

The present invention also provides a pharmaceutical composition comprising a therapeutically effective amount of a monoclonal antibody that is immunologically reactive to a cell-surface glycoprotein that is CD44 and that is able to inhibit OKT3-induced T cell proliferation and in a pharmaceutically acceptable carrier. A preferred embodiment of this invention provides the monoclonal antibody mAb 212.3 in a pharmaceutically acceptable carrier.

The present invention provides a method for immunosuppression in a mammal comprising administering a therapeutically effective amount of a monoclonal antibody or fragment thereof that is immunologically reactive to a cell-surface glycoprotein that is CD44 wherein the monoclonal antibody is able to inhibit OKT3-induced T cell proliferation and in a pharmaceutically acceptable carrier. In a preferred embodiment of the method the mammal is a human. Another preferred embodiment provides mAb 212.3 for use in this method.

The present invention provides a method for treating autoimmune disease in a mammal comprising administering a therapeutically effective amount of a monoclonal antibody or fragment thereof that is immunologically reactive to a cell-surface glycoprotein that is CD44 and that is able to inhibit OKT3-induced T cell proliferation and in a pharmaceutically acceptable carrier. In a preferred embodiment of the method the mammal is a human. Another preferred embodiment provides mAb 212.3 for use in this method.

The present invention provides a method for treating human organ transplant recipients comprising administering a therapeutically effective amount of a monoclonal antibody or fragment thereof that is immunologically reactive to a cell-surface glycoprotein that is CD44 wherein the monoclonal antibody is able to inhibit OKT3-induced T cell proliferation and in a pharmaceutically acceptable carrier. Another preferred embodiment provides mAb 212.3 for use in this method.

It is a further object of the present invention to provide an epitope of a cell surface glycoprotein wherein the epitope is immunologically reactive to an antibody and wherein the antibody is characterized on the basis that it inhibits OKT3-induced cellular proliferation. In a preferred embodiment, the epitope is present on the surface of a T cell. In another preferred embodiment the glycoprotein is CD44. In another preferred embodiment the monoclonal antibody is mAb 212.3.

It is another object of the invention to provide a chimeric antibody that is immunologically reactive to a cell-surface glycoprotein that is CD44. It is a particular object of the invention to provide such an antibody which inhibits OKT3-induced T cell proliferation. In a preferred embodiment, the chimeric antibody is a monoclonal antibody.

Further objects and preferred embodiments of the present invention will be discussed in the following description of the preferred embodiments and claims.

## DETAILED DESCRIPTION OF THE PREFERRED EMBODIMENTS

The present invention encompasses antibodies that are immunologically reactive with an epitope of a glycoprotein that is CD44 on cells, including T cells, and that are able to inhibit OKT3-induced T cell proliferation. The invention specifically encompasses monoclonal antibodies and hybridoma cell lines that produce such antibodies. The epitope that is immunologically reactive with the antibodies provided by the invention is also within the scope of the invention. In addition, methods of using the antibodies of the invention for therapeutic uses are included within the scope of the claimed invention.

The antibodies provided by the invention can be raised in animals by inoculation with cells that express CD44 molecules or epitopes of the CD44 molecule using methods well known in the art. Animals that can be used for such inoculations include individuals from species comprising cows, sheep, pigs, mice, rats, rabbits, hamsters, goats and primates. Preferred animals for inoculation are rodents (including mice, rats, hamsters) and rabbits. The most preferred animal is the mouse.

Cells that can be used for such inoculations, or for any of the other means used in the invention, include human T cells, human T cell lines, or any cell line which naturally expresses CD44, or any cell or cell line that expresses CD44 or any epitope therein as a result of molecular or genetic engineering, or that has been treated to increase the expression of CD44 by physical, biochemical or genetic means. Preferred cells are human cells, more preferably human T cells, and the most preferred human T cells are pokeweed mitogen-stimulated suppressor T cells from human peripheral blood, generated and characterized as described by Aune and Pogue, 1989, J. Immunol. 142: 3731.

The present invention provides monoclonal antibodies that are immunologically reactive with an epitope that is CD44 present on the surface of human cells and that are able to inhibit OKT3-induced T cell proliferation. These antibodies are made using methods and techniques well known to those of skill in the art.

Monoclonal antibodies provided by the present invention are produced by hybridoma cell lines, that are also provided by the invention and that are made by methods well known in the art. Hybridoma cell lines are made by fusing individual cells of a myeloma cell line with spleen cells derived from animals immunized with cells expressing CD44, including human cells, as described above. The myeloma cell lines used in the invention include lines derived from myelomas of mice, rats, hamsters, primates and humans. Preferred myeloma cell lines are from mouse, and the most preferred mouse myeloma cell line is P3X63-Ag8.653. The animals from whom spleens are obtained after immunization are rats, mice and hamsters, preferably mice, most preferably Balb/c mice. Spleen cells and myeloma cells are fused using a number of methods well known in the art, including but not limited to incubation with inactivated Sendai virus and incubation in the presence of polyethylene glycol (PEG). The most preferred method for cell fusion is incubation in the presence of a solution of 45% (w/v) PEG-1450. Monoclonal antibodies produced by hybridoma cell lines can be harvested from cell culture supernatant fluids from *in vitro* cell growth; alternatively, hybridoma cells can be injected subcutaneously and/or into the peritoneal cavity of an animal, most preferably a mouse, and the monoclonal antibodies obtained from blood and/or ascites fluid.

Monoclonal antibodies provided by the present invention can also be produced by recombinant genetic methods well known to those of skill in the art, and the present invention encompasses antibodies made by such methods that are immunologically reactive with an epitope of CD44 and that are able to inhibit OKT3-induced T cell proliferation.

The present invention encompasses fragments of the antibody that are immunologically reactive with an epitope of CD44 and that are able to inhibit OKT3-induced T cell proliferation. Such fragments can be produced by any number of methods, including but not limited to proteolytic cleavage, chemical synthesis or preparation of such fragments by means of genetic engineering technology. The present invention also encompasses single-chain antibodies that are immunologically reactive with an epitope of CD44 and that are able to inhibit OKT3-induced T cell proliferation made by methods known to those of skill in the art.

It is an object of the present invention to provide methods for immunosuppression in a mammal, most preferably a human, comprising administering a therapeutically effective dose of an antibody or fragment of antibody that is immunologically reactive with an epitope of CD44 and that is able to inhibit OKT3-induced T cell proliferation. Routes of administration include but are not limited to oral, intravenous, parenteral, rectal, optical, aural and transdermal.

The present invention also provides a method for treating autoimmune disease in a mammal, most preferably a human, comprising administering a therapeutically effective dose of an antibody or fragment of antibody that is immunologically reactive with an epitope of CD44 and that is able to inhibit OKT3-induced T cell proliferation. Autoimmune diseases intended to be an object of the present invention include but are not limited to systemic lupus erythematosus, rheumatoid arthritis, diabetes, multiple sclerosis and colitis.

It is also an object of the invention to provide a method for treating human organ or tissue transplant recipients comprising administering a therapeutically effective dose of an antibody or fragment of antibody that is immunologically reactive with an epitope of CD44 and that is able to inhibit OKT3-induced T cell proliferation. Recipients intended to benefit from such a method are humans who have received transplanted organs or tissues, including but not limited to the following organs or tissues: heart, lung, liver, kidney, pancreas, skin, bone marrow, bone, cornea and hair.

The present invention also encompasses an epitope of CD44 that is comprised of sequences and/or a conformation of sequences present in the CD44 molecule. This epitope is immunologically reactive to mAb 212.3. This epitope may be naturally occurring, or may be the result of proteolytic cleavage of the CD44 molecule and isolation of an epitope-containing peptide or may be obtained by synthesis of an epitope-containing peptide using methods well known to those skilled in the art. The present invention also encompasses epitope peptides produced as a result of genetic engineering technology and synthesized by genetically engineered prokaryotic or eukaryotic cells.

The invention also includes chimeric antibodies, comprised of immunologically reactive light chain and heavy chain peptides to an epitope on the cell surface of T cells that is CD44, wherein such chimeric antibodies are able to inhibit OKT3-induced T cell proliferation. The chimeric antibodies embodied in the present invention include those that are derived from naturally occurring antibodies as well as chimeric antibodies made by means of genetic engineering technology well known to those of skill in the art.

The following examples describe certain specific embodiments of the invention. However, many additional embodiments not described herein nevertheless fall within the spirit and scope of the present invention and claims.

The hybridoma which is producing the mAb 212.3 has been deposited on January 29, 1992 under Budapest Treaty with the American Type Culture Collection, 12301 Parklawn Drive, Rockville, MD 20852 USA. The cell has been provided with the ATCC Designation HB 10970.

## EXAMPLE 1

### Preparation of Anti-CD44 Monoclonal Antibodies

Monoclonal antibodies (mAb) such as mAb 212.3 were prepared by standard methods. Mice were immunized by intradermal injection of $1 \times 10^7$ pokeweed mitogen-stimulated suppressor T cells from human peripheral blood, generated and characterized as described by Aune and Pogue, 1989, J. Immunol. 142: 3731. The immunized mice were sacrificed 3 days after their last injection and their spleens were removed. Single cell suspensions of spleen cells were prepared from the immunized mice and then $1 \times 10^8$ spleen cells were mixed with $5 \times 10^7$ myeloma cells from the non-immunoglobulin secreting mouse myeloma cell line P3X63-Ag8.653, obtained from the American Type Culture Collection (ATCC, Rockville, MD, available as accession no. CRL 1580), and cell suspensions were prepared in serum-free RPMI-1640 culture media (GIBCO, Grand Island, NY). The cells were fused by the dropwise addition of 100 $\mu$l of prewarmed 45% (w/v) PEG-1450 (Eastman Kodak Co., Rochester, NY) to a total volume of 1.0 ml in 1 minute and subsequent dilution with 10 ml serum-free RPMI-1640 culture media. After fusion, individual wells of 24-well tissue culture places (Costar, Cambridge, MA) were seeded with $1 \times 10^6$ cells. Hybrid cells were selected using hypoxanthine-aminopterinthymidine (HAT) supplemented culture medium. Hybridoma cells lines secreting antibodies were cloned at least twice by the limiting dilution method using culture medium supplemented with 5 units/ml human recombinant IL-6 (Genzyme, Boston, MA). Clones were screened and selected by their ability to inhibit suppression of T cell proliferation in a T suppressor cell assay (Aune and Pogue, *supra*). One anti-CD44 mAb obtained from this procedure, designated mAb 212.3, is of the IgG$_{2\alpha}$ isotype. Purified mAb 212.3 was isolated from mouse ascites fluid using protein A-Sepharose CL-4B beads (Pharmacia LKB Biotechnology, Inc., Piscataway, NJ).

## EXAMPLE 2

### Characterization of Anti-CD44 mAbs by Immunoprecipitation and Peptide Mapping

Monoclonal antibodies prepared according to Example 1, including mAb 212.3, may be characterized by immunoprecipitation and peptide mapping. The pattern of T cell surface antigens immunoprecipitated with mAb 212.3 was compared with that of a known anti-CD44 antibody BU52 (obtained from The Binding Site, Ltd., Birmingham, England) to determine if mAb 212.3 was specific for the CD44 molecule. Peptide mapping was then used to compare the peptide profiles of the T cell-specific cell-surface peptides generated by proteolytic cleavage of proteins precipitated with mAb 212.3 with the peptide profiles generated in a similar manner from proteins precipitated by the known anti-CD44 antibody BU52.

PBL were obtained from the buffy coat of whole blood from normal human volunteers. After isolation on Lymphoprep gradients (Nycomed AS, Oslo, Norway), PBL were washed twice in Dulbecco's Phosphate Buffered Saline (D-PBS), suspended in a solution of 10% dimethylsulfoxide (DMSO) and 90% heat-inactivated fetal bovine serum (FBS) (Armour Pharmaceutical Company, Kankakee, IL) and stored in liquid nitrogen until use. Cell surface antigen and proliferative responses of PBL after storage in liquid nitrogen were similar to those properties of fresh lymphocytes.

T cells were isolated from PBL by E-rosetting techniques as described by Blue *et al.*, 1986, J. Immunol. 137: 1202. T cell surface proteins were then labeled with $^{125}$I (Amersham, Arlington Heights, IL) by the lactoperoxidase method and solubilized as described by Blue *et al.*, 1988, Cell 54: 413. Briefly, $^{125}$I-labeled T cells were solubilized in a detergent lysis buffer [0.15 M NaCl, 1 mM EDTA, 10 mM Tris-HCl (pH 8.8), 1 mM CaCl$_2$, 1% NP-40] for 30 minutes at 4°C. The supernatants from the detergent solubilized cells were then precleared by two rounds of incubation with crude protein A (Sigma Chemical Co., St. Louis, MO) and washed once with protein A-Sepharose CL-4B beads (Pharmacia LKB Biotechnology, Inc., Piscataway, NJ). After preclearing, the supernatants were incubated at 4°C for 18 hours with gentle agitation with the desired monoclonal antibodies and protein A-Sepharose CL-4B beads to which rabbit anti-mouse monoclonal antibodies (Jackson Immunological Research Lab, West Grove, PA) had been bound. Protein A beads were then washed once with a high salt buffer [0.4 M NaCl, 5 mM EDTA, 50 mM Tris-HCl (pH 8.0), 1% NP-40, 0.1% sodium dodecylsulfate (SDS), 0.05% sodium deoxycholate] and twice with the detergent lysis buffer to liberate antibody-bound protein.

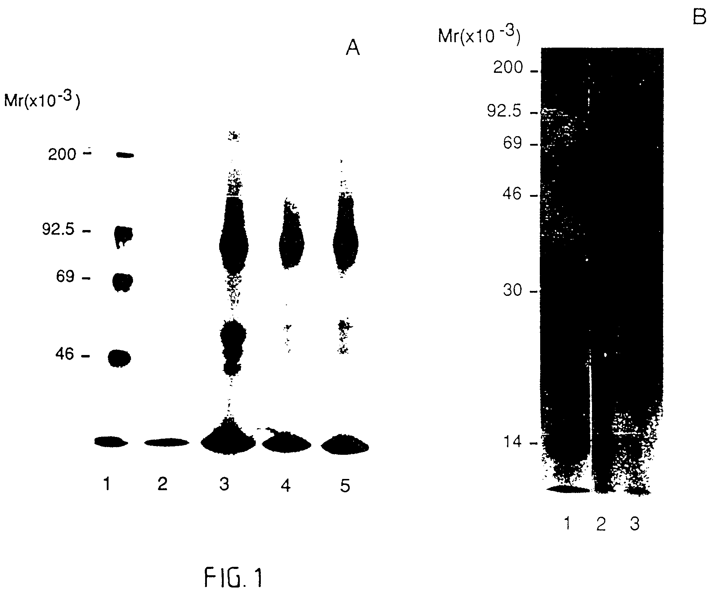

$^{125}$I-labeled proteins immunoprecipitated as described above were analyzed by gel electrophoresis on 7.5% SDS polyacrylamide slab gels under the reducing conditions as described by Laemmli, 1970, Nature 220: 680. After electrophoresis, gels were fixed and vacuum-dried, and radiolabeled proteins were visualized by autoradiography on Kodak XAR-5 film (Eastman Kodak Co., Rochester, NY). In addition to mAb 212.3, control antibodies used included a mouse IgG$_{2\alpha}$ (UPC10; obtained from Sigma Chemical Co., St. Louis, MO) and the anti-CD44 mAb BU52. An example of the results of autoradiography of proteins immunoprecipitated in this way is shown in Figure 1. As shown in Figure 1A, mAb 212.3 (lanes 4 and 5) and the known CD44-specific mAb BU52 (lane 3) both recognize a major 80-to-90 kD molecule on $^{125}$I-labeled T cells. Molecular weight standards are shown in lane 1, and the results of a control immunoprecipitation using protein A alone are shown in lane 2.

The identity of the T cell surface antigens recognized by mAbs 212.3 and BU52 was further demonstrated by peptide mapping using the proteolytic enzyme chymotrypsin at a concentration of 2 µg/ml as described by Blue *et al.*, 1988, *supra*. The peptide products of chymotrypsin digestion of the CD44 protein immunoprecipitated by either mAb 212.3 or BU52 were then separated by polyacrylamide gel electrophoresis and visualized by autoradiography as described above. An example of such peptide mapping results are shown in Figure 1B. As shown in Figure 1B, chymotrypsin digestion of immunoprecipitates prepared with the mAb 212.3 (lane 2) and the anti-CD44 mAb BU52 (lane 3) result in virtually identical peptide profiles, further demonstrating the specificity of mAb 212.3 as an anti-CD44 mAb.

## EXAMPLE 3

### Analysis of the Effect of Anti-CD44 mAbs on Human Hematological Cells and Cell Lines by Flow Microfluorimetry

Monoclonal antibodies prepared according to Example 1, including mAb 212.3, may be further characterized by competitive binding studies and flow microfluorimetry (FMF). PBL were isolated as described in Example 2. Aliquots of 1 x 10$^6$ cells were washed twice with PBS containing 0.2% human serum and incubated with fluorochrome-conjugated specific antibody or unconjugated specific antibody at saturating concentrations for 30 minutes at 4°C and then washed three times with PBS/0.2% human serum. For indirect immunofluorescence, cells were stained with fluorescein isothiocyanate (FITC)-conjugated goat anti-mouse IgG (Cappel Labs, Organon Teknika Corp., West Chester, PA) for 30 minutes at 4°C after incubation with unconjugated specific antibodies. FMF was performed on a Becton Dickinson FACScan flow cytometer using forward and orthogonal light scatter to select viable, intact cells. Fluorescence was measured using four decade logarithmic amplification. Data for 10,000 cells were collected in list mode in a CONSORT 30 computer and subsequently analyzed using LYSIS software. Linear comparisons of fluorescence intensity measurements obtained with cells stained with anti-CD44 mAbs 212.3 or BU25 were determined as described by Schmid *et al.*, 1988, Cytometry 9: 533. Briefly, a Delta Mean Channel (DMC) value for each sample was determined by subtracting background fluorescence as measured by the mean channel density (MCD) of cells that had been incubated with a non-specific control antibody (UPC10) from MCD of cells stained after incubation with anti-CD44 specific antibodies. DMC values were then converted to a log unit (Y) by dividing the DMC by the number of channels per decade. Relative linear fluorescence values were determined by taking the antilog of Y ($10^Y$).

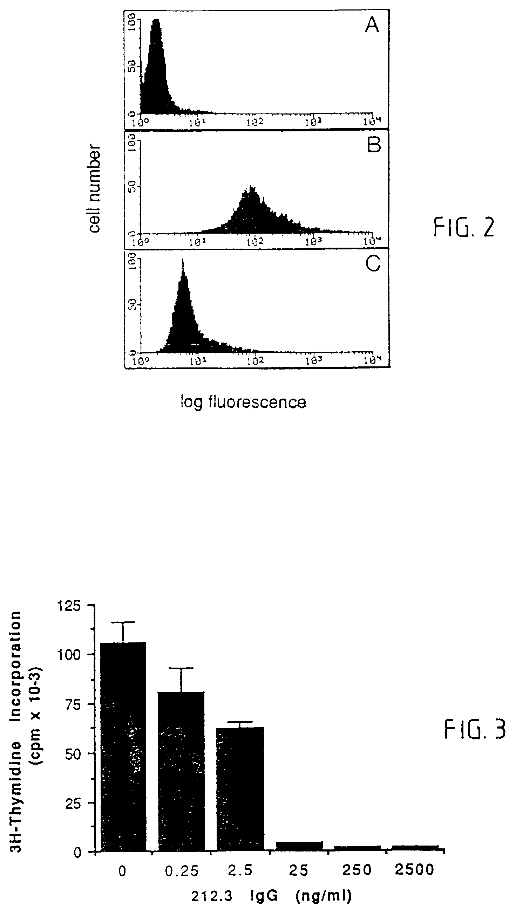

Direct binding as well as competitive binding experiments analyzed by FMF as described above were performed using mAbs 212.3, BU52 and OKT3. OKT3 hybridoma cells (available from the ATCC as accession no. CRL 8001) were grown in Balb/c mice to produce ascites fluid that was the source of OKT3 mAb. An example of the results of experiments with mAb 212.3 and BU52 are shown in Figure 2 (A,B,C). Figure 2A shows the background fluorescence pattern by FMF analysis plotted as cell number versus log fluorescence displayed by unstained PBL (not incubated with anti-CD44 antibody). Figure 2B shows the positive fluorescence staining pattern of cells directly stained with FITC-conjugated mAb 212.3. Figure 2C shows that the positive fluorescence pattern of cells stained with FITC-conjugated mAb 212.3 as shown in Figure 2B can be completely inhibited by pre-incubation with saturating amounts of the anti-CD44-specific antibody BU52 under the conditions described above. The inhibition of the binding of mAb 212.3 to T cells by pre-incubation with BU52 may indicate that the epitopes recognized by these antibodies are overlapping or in proximity to one another. However, other differences in the properties of these antibodies clearly demonstrate that the antibodies recognize different and distinct epitopes.

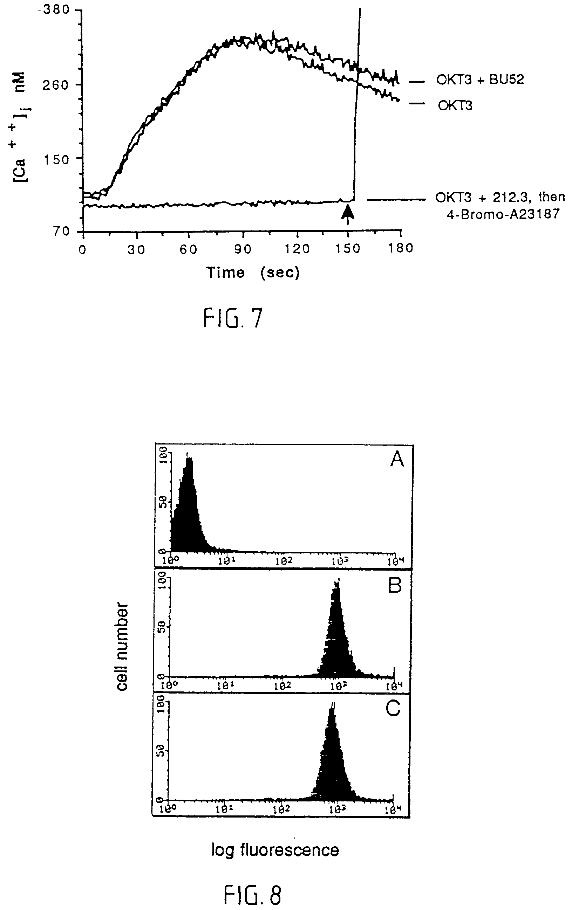

No inhibition was observed when PBL were pre-incubated with another T cell specific mAb, the anti-CD3-specific mAb OKT3. The results of these experiments are shown in Figure 8 (A,B,C). Figure 8A shows the background fluorescence pattern displayed by unstained PBL. Figure 8B shows the positive fluorescence staining pattern of cells incubated with biotin-conjugated OKT3 IgG and stained with phycoerythrin (PE)-conjugated streptavidin. Figure 8C shows that the positive fluorescence staining pattern of cells incubated with biotin-conjugated OKT3 IgG and PE-conjugated streptavidin as shown in Figure 8B is unaffected by pre-incubation with saturating amounts of mAb 212.3 IgG (2.5 $\mu$g/ml) for 30 minutes at 4°C. Thus, pre-incubation with mAb 212.3 has no inhibitory effect on the binding of OKT3 to PBL.

The CD44 expression pattern as detected by the binding of mAb 212.3 and BU52 to a variety of human hematological cells and cell lines was also investigated by FMF analysis. Results of representative experiments are summarized in Table I. For these experiments, CD44 expression was determined by labeling cells with mAb 212.3, BU52 or UPC10 (as an IgG control) and FITC-conjugated goat anti-mouse IgG. The data from the FMF analysis of stained cells shown in Table I were expressed as a linear comparison of fluorescence intensity as described above. For these experiments, the human cell lines Ramos, K562 and Jurkats were obtained from ATCC (available as accession nos. CRL 1596, CCL 243, and CRL 8163, respectively) and maintained in RPMI 1640 supplemented with 10% heat-inactivated FBS, 1 mM L-glutamine and 50 $\mu$M $\beta$-mercaptoethanol ($\beta$ME).

Table I

| FMF Analysis of CD44 Expression | | | |
|---|---|---|---|
| Cells | mAb[a] | | |
| | 212.3 | BU52 | UPC10 |
| PBL | 77.2 | 76.6 | 1 |
| Resting T cells | 177.7 | 165.4 | 1 |
| Activated T cells | 100.1 | 100.8 | 1 |
| Jurkats | 0.99 | 1.0 | 1 |
| RBC | 20.1 | 26.5 | 1 |
| Ramos | 1.04 | 1.01 | 1 |
| K562 | 1.5 | 1.07 | 1 |

[a]Data are expressed as a linear comparison of fluorescence intensities as described above.

The results shown in Table I demonstrate that mAb 212.3 has a characteristic pattern of reactivity similar to the pattern seen with the anti-CD44 mAb BU52. The epitope recognized by mAb 212.3 was also found on a variety of other human cell lines, including U937, F-11 and MRC-5.

## EXAMPLE 4

### Analysis of Anti-CD44 mAb Activity by Lymphocyte Proliferation Assays

Monoclonal antibodies prepared according to Example 1, including mAb 212.3, may be further characterized using a lymphocyte proliferation assay. PBL were obtained as described in Example 2. PBL were cultured at 1 x 10$^6$ cells/ml in complete medium consisting of RPMI 1640, 10% heat-inactivated FBS, 1 mM L-glutamine, and 50 $\mu$M $\beta$ME for 3-7 days in 96-well microculture plates (Becton Dickinson Labware, Lincoln Park, NJ) in a humidified atmosphere of 5% CO$_2$ in air at 200 $\mu$l/well. PBL were then stimulated to proliferate by culturing the cells in complete media that was supplemented with either phytohemagglutinin (PHA; 1/80 dilution), anti-CD3 specific mAb OKT3 (1/1000 dilution) or pokeweed mitogen (PWM; 1/500 dilution) for 3, 3, or 7 days, respectively, in the presence or absence of anti-CD44 antibody mAb 212.3 or BU52.

For mixed lymphocyte reaction (MLR) assays, PBL at a concentration of $2 \times 10^7$ cells/ml were incubated with 40 $\mu$g/ml mitomycin C (Sigma Chemical Co., St. Louis, MO) in RPMI 1640/10% FBS media at 37°C for 30 minutes, and then washed with fresh RPMI 1640/10% FBS media 3 times to remove mitomycin C. The mitomycin C-treated PBL ($5 \times 10^5$ cells/ml) were then incubated with untreated allogeneic PBL ($1.5 \times 10^6$ cells/ml) in complete media (described above) for 5 days in 96-well microtitre plates in a total volume of 200 $\mu$l under a humidified atmosphere of 5% $CO_2$ in air. Monoclonal antibody preparations containing azide were dialyzed to remove the azide before use and added at the initiation of each 5-day cell culture.

Incorporation of [$^3$H]-thymidine (TdR) was used as a measure of the degree of cell proliferation induced by the various mitogens, antibodies or allogeneic cells. To measure [$^3$H]TdR incorporation, 1 $\mu$Curie ($\mu$Ci) of [$^3$H]TdR (2 Ci/mmol, Amersham) was added to triplicate cultures 6 hours before cells were harvested onto filter paper (PHD Cell Harvester, Cambridge, MA). Data from such experiments are expressed as the average [$^3$H]TdR incorporation in triplicate samples; the standard deviation of these measurements is less than 10%.

Results of representative lymphocyte proliferation assays showing the effect of anti-CD44 mAb on T cell proliferation are found in Table II.

Table II

| Effect of Anti-CD44 mAb on T Cell Proliferation | | | |
|---|---|---|---|
| Stimulus | Media[a] | mAb | |
| | | 212.3 | BU52 |
| PHA | 155,325 | 141,184 | 134,140 |
| PWM | 102,992 | 86,908 | 117,223 |
| OKT3 | 117,939 | 1,268 | 107,552 |
| MLR | 19,478 | 20,191 | ND |

[a]Values in this column are under conditions of cell culture in the prescence of stimulus but without the addition of monoclonal antibody.

As illustrated in Table II, incubation of PBL with mAb 212.3, in contrast to incubation with BU52, completely inhibits anti-CD3 mAb OKT3-induced T cell proliferation. Thus, the specificity of mAb 212.3 can be distinguished from other anti-CD44 antibodies, including BU52, on this basis. As also shown in Table II, mAb 212.3 has no inhibitory effect on the phytohemagglutinin or pokeweed mitogen mediated stimulation of T cell proliferation. The results of further studies shown in Figure 3 demonstrated that mAb 212.3 inhibits [$^3$H]TdR incorporation of OKT3-stimulated T cells by greater than 95% at concentrations as low as 25 ng/ml; 50% inhibition levels are seen at mAb 212.3 concentrations of 2.5 ng/ml. However, the addition of mAb 212.3 either 24 or 48 hours after initiation of cell culture had no effect on OKT3-stimulated T cell proliferation.

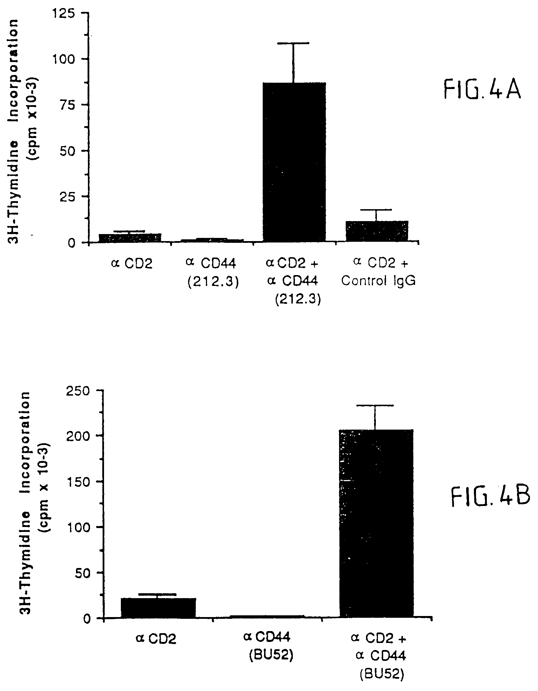

The effect of incubating PBL with mAb 212.3 or BU52 on CD2-receptor-mediated T cell activation as measured by anti-CD2 mAb stimulation was also investigated. Cells were cultured as described above with and without anti-CD44 mAb 212.3 or BU52 and T cell proliferation was stimulated by the addition of suboptimal amounts of two anti-CD2 mAbs directed against the epitope TII$_2$ and the epitope TII$_3$ (Meuer *et al.*, 1984, Cell 36: 897). Results of a representative experiment are shown in Figure 4 (A,B). An approximately 10-fold increase in [$^3$H]TdR incorporation was found when PBL were stimulated with anti-CD2 mAb in the presence of BU52 (Figure 4B). In contrast to the inhibition of OKT3-induced T cell proliferation by mAb 212.3, an approximately 10-fold increase in [$^3$H]TdR incorporation was found when PBL were stimulated with anti-CD2 mAb in the presence of mAb 212.3 (Figure 4A). Figure 4 (A,B) also shows that neither BU52 nor mAb 212.3 alone stimulate proliferation of unstimulated T cells (i.e., in the absence of anti-CD2 antibody stimulation).

## EXAMPLE 5

**Inhibition of IL-2 Release and Expression of the IL-2 Receptor by Anti-CD44 mAb**

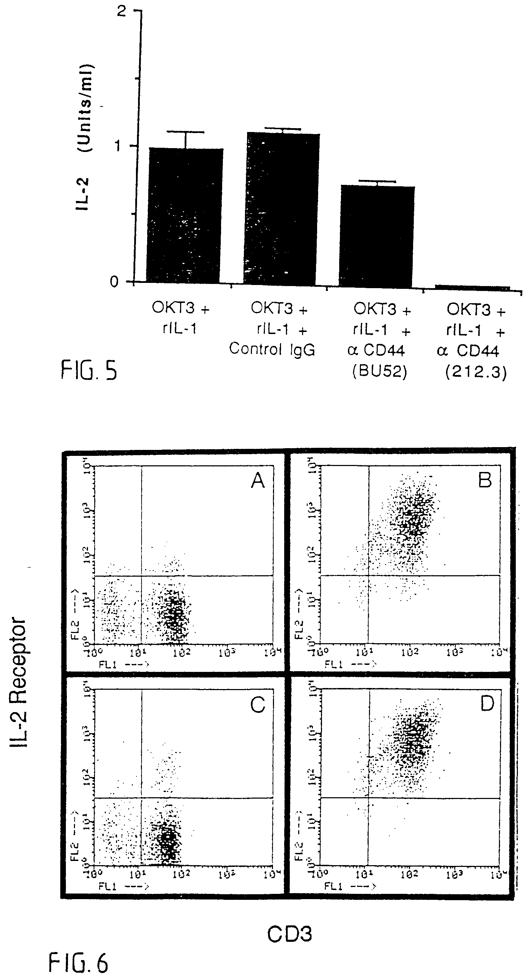

Monoclonal antibodies prepared according to Example 1, including mAb 212.3, may inhibit two essential T cell proliferation-associated events: production of the cytokine interleukin 2 (IL-2) and expression of IL-2 receptors (IL-2R) on the T cell surface. For IL-2 production assays, PBL (2 x $10^6$ cells/ml) in complete medium were cultured in flat bottom 24-well plates for 24 hours, in the presence of anti-CD44 mAb 212.3 (2.5 $\mu$g/ml) or BU52 (1/1000), or UPC10 (2.5 $\mu$g/ml,IgG control) and mAb OKT-3 (1/1000) along with recombinant interleukin 1$\alpha$ at 100 U/ml (rIL-1$\alpha$; Boehringer Mannheim Biochemicals, Indianapolis, IN). Cell culture fluids were obtained at various times and assayed for IL-2 activity by measuring proliferation of an IL-2-dependent murine T cell line HT-2 (Watson, 1979, J. Exp. Med. 150: 1510). The mAb 212.3 had no effect on the ability of the HT-2 cell line to proliferate in response to IL-2. HT-2 cell proliferation stimulated by purified recombinant IL-2 (rIL-2; Boehringer Mannheim Biochemicals) was used as a standard, and one unit of rIL-2 activity was defined as the amount required to cause 50% of maximal stimulation in a 24 hour assay. Measurement of IL-2 levels in 24 hour culture fluids obtained from OKT3-stimulated PBL cultures demonstrated that the addition of mAb 212.3 completely inhibits the production of IL-2, whereas the addition of the anti-CD44 mAb BU52 has no significant effect on OKT3-mediated IL-2 release, as shown in Figure 5.

Cell-surface expression of IL-2R and the effect of anti-CD44 mAb on IL-2R expression on T cells stimulated with OKT3 was determined by FMF analysis (see Example 3). PBL were cultured in complete media as described in Example 4 in the presence of the mAb OKT3 for 3 days in the presence or absence of mAbs 212.3 or BU52. Cells were then stained with FITC-conjugated anti-CD3 antibody (FL1 in Figure 6) and PE-conjugated anti-CD25 antibody (FL2 in Figure 6) and analyzed by FMF. Results of a representative experiment are presented in Figure 6. Figure 6 shows the anti-CD3 (FL1) and anti-CD25 (FL2) staining of resting PBL cultured for 3 days (Figure 6A); PBL cultured with OKT3 mAb for 3 days (Figure 6B); PBL cultured with OKT3 mAb and mAb 212.3 for 3 days (Figure 6C); and PBL cultured with OKT3 mAb and BU52 for 3 days (Figure 6D). As shown in Figure 6, mAb 212.3 completely blocked OKT3 induced expression of IL-2R on T cells, whereas the reference anti-CD44 mAb BU52 had no effect.

## EXAMPLE 6

**Inhibition by Anti-CD44 mAb of Intracellular Calcium Ion Increase in Proliferation-Stimulated T Cells**

A rapid increase in intracellular calcium ion ($[Ca^{++}]_i$) levels is one response during T cell proliferation stimulated by incubation with anti-CD3 antibodies. This event is among the earliest biochemical responses detected, and has been proposed to be an important mechanism for signal transduction from the cell surface to the interior of the cell. Pre-incubation of OKT-3-stimulated T cells with monoclonal antibodies prepared according to Example 1, including mAb 212.3 may block this increase in $[Ca^{++}]_i$ concentration.

Mobilization of $[Ca^{++}]_i$ was evaluated by the INDO-1 method (Grynkiewicz *et al.*, 1985, J. Biol. Chem. 260: 3440; Rabinovitch and June, 1990, in *Flow Cytometry and Sorting*, Melamed *et al.*, eds., Wiley-Liss, NY, p.651) using the $Ca^{++}$ ion-specific fluorescent dye INDO-1 (Molecular Probes, Junction City, OR) and a modified FACStar Plus flow cytometer (Kelley, 1989, Cytometry 10: 796). Briefly, cells were incubated with 4 $\mu$M INDO-1 in Dulbecco's modified Eagle's media (DMEM) containing 10 mM 4-(2-hydroxyethyl)-1-piperazineethanesulfonic acid (HEPES) buffer for 45 minutes at 37°C, washed three times and resuspended in D-PBS at 1 x $10^6$ cells/ml. Aliquots (1 ml) were pre-incubated with D-PBS, or anti-CD44 mAb [212.3 (2.5 $\mu$g/ml) or BU52 (1/1000)] for 5 minutes. The cells were then placed in the modified sample station of the flow sorter, stable flow was achieved, and data acquisition was initiated. Approximately 5 seconds after data collection began, OKT3 antibody (1/500) was added directly to the sample, and data acquisition continued for up to 3 minutes. As a control, the addition of mAb 212.3 (2.5 $\mu$g/ml) or mAb BU52 (1/1000) directly to INDO-1-labeled PBL did not induce detectable changes in $[Ca^{++}]_i$ levels. An argon laser emitting coherent light of wavelength 488 nm at 200 mW generated scatter signals used to gate on viable, intact cells for analysis. A second argon ion laser emitting at wavelengths of 351/364 nm at 100 mW was used to excite the INDO-1 dye. Fluorescence emission was divided by a long pass filter (455 nm cutoff) placed at 45° to the collecting lens and measured simultaneously by two photomultiplier tubes, one equipped with a 20 nm bandpass centered at 395 nm and the other with a 22 nm bandpass centered at 485 nm (Omega Optical, Brattelboro, VT). Data collected in list mode were subsequently processed to yield a ratio of violet fluorescence (395/20 nm) to blue fluorescence (485/22 nm) as a function of time. Calcium concentration was calculated using the formula:

$$[Ca^{++}]_i = K_d \times \frac{(R-Rmin)}{(Rmax-R)} \times \frac{Sf2}{Sb2}$$

where $K_d$ is the INDO-1/$Ca^{++}$ dissociation constant (250 nM); R is the violet/blue fluorescence ratio; $R_{min}$ is the ratio of INDO-1 loaded cells in the absence of $Ca^{++}$ (obtained in the presence of 1 mM $MnCl_2$); $R_{max}$ is the ratio of INDO-1 loaded cells at saturating $Ca^{++}$ levels (obtained in the presence of 5 $\mu$M 4-Br-A23187; Molecular Probes, Junction City, OR); and $S_{f2}/S_{b2}$ is the ratio of the blue fluorescence of the calcium-free and calcium-bound dye (Grynkiewicz *et al.*, *supra*).

Results of representative experiments are shown in Figure 7. Results in Figure 7 show that mAb 212.3 completely inhibits OKT3-mediated increases in intracellular $[Ca^{++}]_i$. Specifically, in control T cells (i.e., in the absence of anti-CD44 mAb), addition of OKT3 results in the elevation of intracellular $[Ca^{++}]_i$ from 110 nM to 300 nM within 1.5 minutes. Addition of the anti-CD44 mAb BU52 has no effect on this increase in $[Ca^{++}]_i$. In contrast, addition of mAb 212.3 completely inhibits this elevation of $[Ca^{++}]_i$ levels. This result indicates that mAb 212.3 binding to its T cell surface epitope is able to affect intracellular biochemical events that accompany anti-CD3 mediated T cell proliferation. The inhibition by mAb 212.3 can be overridden, and increases in intracellular $[Ca^{++}]_i$ levels occur, when the cells are exposed to the calcium ionophore 4-Br-A23187. It appears that the mAb 212.3 mediated inhibition of OKT3-stimulated increases in intracellular $[Ca^{++}]_i$ levels does not result simply from blocking the binding of OKT3 to T cells. This was shown by the competitive antibody binding experiments described in Example 3 and shown in Figure 8. Nonetheless, as shown in Figure 7 above, the mAb 212.3 is able to inhibit OKT3-stimulated increases in intracellular $[Ca^{++}]_i$ levels.

**Claims**

1.  An antibody or fragment thereof that is immunologically reactive to CD44, wherein the antibody inhibits OKT3-induced T cell proliferation.

2.  An antibody according to claim 1, wherein the antibody is a monoclonal antibody.

3.  A cell line which produces an antibody or fragment thereof that is immunologically reactive to CD44, wherein the antibody inhibits OKT3-induced T cell proliferation.

4.  A cell line according to claim 3, wherein the antibody is a monoclonal antibody.

5.  A pharmaceutical composition comprising a therapeutically effective amount of an antibody or fragment thereof according to claim 1 in a pharmaceutically acceptable carrier.

6.  An epitope of CD44 wherein the epitope is immunologically reactive to the antibody or fragment thereof according to claim 1.

7.  An epitope according to claim 6 which is present on the cell-surface of a T cell.

8.  A chimeric antibody that is immunologically reactive to CD44, wherein the antibody inhibits OKT3-induced T cell proliferation.

9.  Use of the antibody according to claims 1 and 2 in the preparation of pharmaceuticals.

FIG. 1

FIG. 2

FIG. 3

FIG.4A

FIG.4B

FIG. 5

IL-2 Receptor

CD3

FIG. 6

15

FIG. 7

log fluorescence

FIG. 8

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| D,X | EP-A-0 322 254 (BECTON DICKINSON AND COMPANY) <br> * the whole document * <br> --- | 1-9 | C12P21/08 <br> C12N5/20 <br> A61K39/395 |
| P,X | THE JOURNAL OF IMMUNOLOGY <br> vol. 147, no. 8, 15 October 1991, BALTIMORE MD, USA <br> pages 2493 - 2499; <br> B. ROTHMAN ET AL.: 'Human T cell activation by OKT3 is inhibited by a monoclonal antibody to CD44.' <br> * abstract * <br> --- | 1-9 | C07K15/00 |
| A | PROCEEDINGS OF THE NATIONAL ACADEMY OF SCIENCES OF USA <br> vol. 86, no. 24, December 1989, WASHINGTON DC, USA <br> pages 10029 - 10033; <br> C. QUEEN ET AL.: 'A humanized antibody that binds to the interleukin-2 receptor.' <br> * abstract * | 8 | |

TECHNICAL FIELDS
SEARCHED (Int. Cl.5)

C12P
C12N
A61K
C07K

The present search report has been drawn up for all claims

| Place of search | Date of completion of the search | Examiner |
|---|---|---|
| THE HAGUE | 13 MAY 1992 | NOOIJ F.J.M. |